# EUROPÄISCHE PATENTSCHRIFT

(11) **EP 3 565 639 B1**
(45) Veröffentlichungstag und Bekanntmachung des Hinweises auf die Patenterteilung: **10.03.2021**
(21) Anmeldenummer: 18701251.3
(22) Anmeldetag: 04.01.2018
(51) Int. Cl.: A61Q 7/00, A61P 17/00, A61K 8/19, A61K 33/00

(54) **HAARWUCHSMITTEL**
HAIR RESTORER
PRODUIT CAPILLAIRE

(30) Priorität: 04.01.2017 DE 102017100121
(43) Veröffentlichungstag der Anmeldung: 13.11.2019
(73) Patentinhaber: Ferk, Franz, 86163 Augsburg (DE)
(72) Erfinder: Ferk, Franz, 86163 Augsburg (DE)
(74) Vertreter: Dr. Klemens Schubert Patentanwalt
(86) Internationale Anmeldenummer: PCT/EP2018/050219
(87) Internationale Veröffentlichungsnummer: WO 2018/127541

(56) Entgegenhaltungen:
- WO-A2-2015/172993
- DE-A1- 3 633 242
- FR-A1- 2 216 987
- US-A1- 2005 087 040
- DATABASE GNPD [Online] MINTEL; 31. Januar 2015 (2015-01-31), Laboratorium Kosmetyczne Dr Irena Eris: "H-Stimupurin Specialist Hair Growth Stimulating Shampoo", XP002778504, Database accession no. 2685751
- DATABASE GNPD [Online] MINTEL; 30. November 2012 (2012-11-30), Isdin Lampdapil: "Anti Hair Loss Shampoo", XP002778505, Database accession no. 1928945
- Madeleine Sommerville: "I dont use shampoo, and havent in over five years", The Guardain, 22 September 2015 (2015-09-22), XP055693200, Retrieved from the Internet: URL:https://www.theguardian.com/lifeandsty le/2015/sep/22/problem-sanctimonious-envir onmentalists-baking-soda [retrieved on 2020-05-08]

## Beschreibung

Die vorliegende Offenbarung betrifft Haarwuchsmittel, bestehend aus einer wässrigen Lösung von Natriumcarbonat, zur Anwendung, wie in den Ansprüchen definiert, mit einem stark alkalischen pH-Wert, die Verwendung des Mittels zur Bekämpfung von Haarausfall sowie ein Verfahren zur Verwendung des Mittels.

Haarwachstum ist kein kontinuierlicher Vorgang, sondern verläuft in Wachstums- und Haarverlustschüben, die sich zyklisch wiederholen. Dieser Vorgang ist im Wesentlichen darauf zurückzuführen, dass Haarfollikelzellen einem genetisch festgelegten Zyklus von Wachstum, Regression und Ruhephase unterliegen. Der Haarfollikel ist damit das einzige menschliche Organ, das sich ständig selbst erneuert und somit einen, in Abhängigkeit von der jeweiligen Wachstumsphase, einzigartigen Metabolismus aufweist. So kommt der Metabolismus des Haarfollikels in der Ruhephase fast völlig zum Erliegen und wird mit jedem neuen Beginn eines weiteren Zyklus ebenfalls neu initiiert. Gesteuert wird dieser Zyklus von einer kleinen, hochspezialisierten Zellpopulation im Haarbulbus, den dermalen Papillenzellen, die durch ein einzigartiges komplexes System molekularer Signale, die spezifisch für jede Phase des Haarzyklus sind, das Haarwachstum kontrolliert. Im Laufe des zyklischen Vorgangs kommt es kontinuierlich zum Ausfall einzelner Haare, wobei ein Haarausfall von bis zu 100 Haaren pro Tag als physiologisch normal bezeichnet wird. Ist der komplexe Metabolismus des Haarfollikels gestört, kann es zum vorzeitigen oder übermäßigen Ausfall der Haare und schließlich zu Alopezie bis hin zu vollständiger Glatzenbildung kommen. Die Ursachen von vorzeitigem oder übermäßigem Haarausfall sind vielfältig und reichen von genetischer Veranlagung, Mangelzuständen, Vergiftungen bis hin zu Infektionen und Haarausfall im Zuge einer Chemotherapie.

Die klassische Form des bisher noch weitgehend als irreversibel einzustufenden Haarausfalls ist der androgenetische Haarausfall (androgenetische Alopezie = AGA, bei ca. 80% der Männer und ca. 40% der Frauen relevant). Er setzt oftmals schon zum Ende der Pubertät (ca. 15-18 Jahre) ein und dürfte im Alter von rund 50-60 Jahren abgeschlossen sein. Nach älteren Studien und Untersuchungen wird der androgenetische Haarausfall vorwiegend durch Androgene (männliche Sexualhormone) ausgelöst. Die zwei wichtigsten biologisch aktiven Formen der Androgene sind das Testosteron und das von ihm abgeleitete 5-α-Dihydrotestosteron (DHT). DHT ist ein Steroidhormon und wird im Körper durch das Enzym 5-α-Reduktase aus Testosteron gebildet. Untersuchungen weisen darauf hin, dass bei androgenetischem Haarausfall eine Überempfindlichkeit der Haarwurzel gegenüber DHT besteht. Es greift den Haarwurzelkomplex an, dieser wird immer stärker geschwächt und der Haarzyklus dadurch nachhaltig gestört. Es werden nicht mehr alle Zyklen (Anagen-, Katagen- und Telogenphase) mit der notwendigen Zeitdauer durchlaufen, mit dem Ergebnis, dass immer mehr Haare ausfallen. Teilweise werden weiter Haare gebildet, wenn auch vermutlich nur noch Vellushaare. Diese marklosen Haare kann man unter bestimmten Lichtverhältnissen gut als Flaum auf den zunehmend kahlen Stellen erkennen. Die Glatzenbildung mit ihren Anfangsformen (Geheimratsecken, Stirnglatze, Tonsur) ist nun unausweichlich. Lediglich der Hinterkopf und Nackenbereich sind unempfindlich gegenüber DHT, was bei Haartransplantationen besonders beachtet wird.

Eine durchaus übliche Möglichkeit dem Problem des Haarausfalls zu begegnen, ist die Verwendung von Haarwuchsmitteln, die topisch aufgebracht werden, z.B. in Form von Lösungen, Tinkturen, Tonika und Lotionen. Diese auf dem Markt zum Teil stark beworbenen Produkte, zeigen nach Tests teilweise nur unbefriedigende Ergebnisse. Überwiegend können diese Mittel nicht während des Schlafs und z.B. während sportlicher Aktivitäten aufgetragen werden, ohne dass der Schlaf bzw. die z.B. sportliche Tätigkeit aktiv unterbrochen wird, so dass nur ein Bruchteil der täglichen maximal möglichen Einwirkzeit von 24 h genutzt werden kann.

Eine weitere Möglichkeit, diese Ursache des Haarausfalls zu bekämpfen, ist der Einsatz von Medikamenten, welche die Umwandlung von Testosteron in DHT im menschlichen Körper hemmen. Als möglicher Wirkstoff hat sich z.B. Finasterid bewährt. Das Mittel muss fortlaufend eingenommen werden, damit der DHT-Spiegel sich nicht wieder erhöht, womit der Haarausfall erneut einsetzen würde. Bei direkten Eingriffen in den jeweiligen Hormonhaushalt des Individuums ist öfter mit nicht vorhersehbaren Nebenwirkungen zu rechnen.
Minoxidil (Lösung oder Schaum) - ein weiterer Haar-Wirkstoff - wird topisch auf die Kopfhaut aufgetragen. Untersuchungen haben gezeigt, dass er allerdings stark toxisch für Katzen sein kann und versehentlicher Hautkontakt für sie bereits zum Tode führen kann.
Nach unabhängigen Tests wirken sich beide Behandlungsansätze (Medikamente mit Finasterid und Minoxidil) in einer nachgelagerten Phase der Verursachungskette nur wenig auf die Durchblutung der Kopfhaut beziehungsweise auf die Anlagerung von DHT aus.

Neuere Erkenntnisse zeigen, dass die AGA möglicherweise umkehrbar ist. Die Hoffnungen auf eine Therapie des männlichen Haarausfalls gründen sich auf den Nachweis von Stammzellen in den Haarfollikeln sowie auf die vermehrte Produktion eines Prostaglandins, dessen Blockade die Stammzellen reaktivieren könnte. Die Haarfollikel sind bei der AGA an den Stellen des Haarausfalls zwar stark verkleinert, sie sind aber noch vorhanden und lebendig. Dort wurden auch Stammzellen nachgewiesen. Seither wird nach einem Aktivator gesucht, der die Follikel wieder zum Leben erwachen und neue Haare sprießen lässt. Dieser Aktivator ist bisher noch nicht gefunden, doch neuere Ergebnisse zeigen, dass der Haarausfall wahrscheinlich nicht die Folge einer genetischen oder durch Hormone bewirkten Erschöpfung ist. Es scheint vielmehr, als würde die Bildung der Haare aktiv durch eine Substanz (Prostaglandin D2) unterdrückt. Eine Neutralisation der Wirkweise dieser Substanz auf die Haarbildung - z.B. durch einen Aktivator - ist bisher noch nicht gefunden worden.

Aus der DE 3633242 A (ARTIRAN, NIHAT) 07.04.1988 ist ein Glatzenbekämpfungsmittel bekannt, welches aus zwei Mischungen besteht, wobei die erste Mischung eine reinigende und das Haarwachstum aktivierende Wirkung auf die Kopfhaut hat und 16 Bestandteile umfasst, und die zweite Mischung nur eine haarwachstumsaktivierende Wirkung hat und zwölf Bestandteile umfasst. Es wird jedoch nicht offenbart, welcher der in den jeweiligen Mischungen vorhandene Bestandteil das Haarwachstum bewirkt oder beeinflusst.

Die FR 2216987 A (SALLES, PIERRE) 06.09.1974 offenbart ein Mittel gegen Alopezie, welches gleichzeitig antiseptisch, antiparasitär und stimulierend wirkt sowie ein Antischuppenmittel enthält. Es wird jedoch ebenfalls nicht offenbart, welcher der in den jeweiligen Mischungen vorhandene Bestandteil das Haarwachstum bewirkt oder beeinflusst.

Ferner ist aus der EP 1940233 B (SOLVAY SA) 09.07.2008 ein Mittel zur Bekämpfung von Kopfläusen bekannt, bei welchem eine mindestens 60%ige Lösung eines Alkalihydrogencarbonats auf das Haar aufgebracht wird. Ein Haarwuchsmittel wird hierin nicht beschrieben.

Aufgabe der vorliegenden Erfindung ist es, ein Haarwuchsmittel zui Anwendung zur Verfügung zu stellen, welches nur wenige Bestandteile aufweist und einfach anzuwenden ist.

Die Aufgabe wird gelöst durch ein Haarwuchsmittel zur Verwendung bei der Förderung oder Verstärkung des Haarwuchses, wie in den Ansprüchen definiert.

Gegenstand der Offenbarung ist ein Haarwuchsmittel, bestehend aus einer wässrigen Lösung eines Alkalisalzes mit einem pH-Wert der Lösung im Bereich von 9,5 bis 12. Im Sinne der vorliegenden Offenbarung wird unter einem Alkalisalz auch eine salzähnliche Verbindung verstanden, welche mindestens ein Alkalimetall enthält.

Bevorzugt ist ein Haarwuchsmittel, wobei der pH-Wert zwischen 9,5 und 11,9 liegt. Weiterhin bevorzugt liegt der pH-Wert zwischen 10 und 11,7. Besonders bevorzugt beträgt der pH-Wert zwischen 10,0 und 11,5.

Bevorzugt ist auch ein Haarwuchsmittel, wobei der Gehalt an Alkalisalz 0,00005 bis 5 Gew.-%, bevorzugt 0,0005 bis 2 Gew.-% und besonders bevorzugt 0,0005 bis 1 Gew.-% beträgt.

Weiterhin offenbart ein Haarwuchsmittel, wobei das Alkalisalz ein Alkalioxid, ein Alkalihydroxid oder ein Alkalicarbonat ist.

Bevorzugt ist auch ein Haarwuchsmittel, wobei das Kation des Alkalisalzes ausgewählt ist aus Li+, Na+ und K+.

Dabei ist weiterhin bevorzugt, dass das Anion des Alkalisalzes ausgewählt ist aus O²⁻, OH⁻, CO₃²⁻ und HCO₃⁻.

Ganz besonders bevorzugt ist dabei, dass das Alkalisalz ausgewählt ist aus Lithiumcarbonat, Natriumcarbonat oder Kaliumcarbonat sowie deren Hydraten.

Offenbart wird auch ein Haarwuchsmittel, wobei das Wasser ausgewählt ist aus der Gruppe enthaltend *aqua purificata,* destillatgleiches Wasser, bidestilliertes Wasser, VE-Wasser oder demineralisiertes Wasser. Unter dem Begriff *aqua purificata* oder "Gereinigtes Wasser" ist im Rahmen der vorliegenden Offenbarung Wasser zu verstehen, das physikalisch gereinigt wurde, jedoch weder absolut steril noch pyrogenfrei sein muss. "Gereinigtes Wasser" ist sowohl "Aqua purificata" gemäß dem Europäischen Arzneibuch ( *Pharmacopoea Europaea,* Ph. Eur.) als auch jedes andere aus Wasser, vorzugsweise Trinkwasser, durch (einfache oder mehrfache) Destillation, lonenaustausch, Umkehrosmose oder andere geeignete Methoden gewonnene Wasser von hoher chemischer und mikrobieller Qualität.

Überraschenderweise wurde festgestellt, dass allein die stark alkalische Eigenschaft des erfindungsgemäßen Haarwuchsmittels für die Stimulierung des Haarwachstums und dem Entgegenwirken bei der Alopezie verantwortlich ist. Es ist daher erforderlich, dem offenbarten Haarwuchsmittel die stark alkalischen Eigenschaften hinzuzufügen. Dies geschieht erfindungsgemäß dadurch, dass man eine wässrige Lösung eines Alkalisalzes oder einer Salzähnlichen Verbindung herstellt. Dies bedeutet, dass geeignete Salze oder salzähnliche Verbindungen ein Alkalikation ausgewählt aus Lithium, Natrium oder Kalium aufweisen, und dass das Anion ausgewählt ist aus Oxiden, Hydroxiden, Carbonaten oder Hydrogencarbonaten (O²⁻, OH⁻, CO₃²⁻ und HCO₃⁻). Geeignet sind daher natriumhaltige Carbonate und Hydrogencarbonate, insbesondere ausgewählt aus Natriumcarbonat (Natrit, Na₂CO₃) und seinen Hydratformen Natriumcarbonathydrat (Thermonatrit, Na₂CO₃·H₂O), Natriumcarbonatheptahydrat (Na₂CO₃·7H₂ O) und Natriumcarbonatdecahydrat (Soda/Kristallsoda, Na₂CO₃·10H₂O), Pirssonit (Dihydrat, Na₂Ca(CO₃)₂·2H₂O), Gaylussit (Pentahydrat, Na₂ Ca(HCO₃)·5H₂O) sowie Trona (Na₃(HCO₃)(CO₃)·2H₂O). Geeignet sind aber auch Alkalisalze, ausgewählt aus Lithiumoxid, Lithiumhydroxid, Lithiumcarbonat, Kaliumcarbonat, Kaliumhydrogencarbonat, Kaliumhydroxid, Natriumoxid, Natriumhydroxid und Mischungen der genannten Alkalisalze.

Zur Herstellung des Haarwuchsmittels wird daher ein entsprechendes Alkalisalz in Wasser gelöst. Die Art des verwendeten Alkalisalzes und die Menge, die in Wasser gelöst wird, führen dann zu dem gewünschten pH-Wert der fertigen wässrigen Lösung. Es beträgt daher der Gehalt an Alkalisalz 0,00005 bis 5 Gew.-%, bevorzugt 0,0005 bis 2 Gew.-% und besonders bevorzugt 0,0005 bis 1 Gew.-%. Die jeweilige zu verwendende Menge des jeweiligen Alkalisalzes kann der Fachmann durch wenige Versuche ermitteln. Damit lässt sich jeder gewünschte pH-Wert einstellen.

Der erfindungsgemäße pH-Wert liegt im Bereich von 9,5 bis 12. Je nach den jeweiligen Erfordernissen, wie beispielsweise der Empfindlichkeit der Haut gegenüber Alkalien, also hohen pH-Werten, kann der Fachmann einen geeigneten pH-Wert auswählen und somit die dafür erforderliche Menge an Alkalisalz in Wasser lösen. Bevorzugte pH-Wertbereiche liegen zwischen 9,5 und 11,9 oder zwischen 10 und 11,7 oder zwischen 10,0 und 11,5.

Gegenstand der vorliegenden Offenbarung ist auch ein Haarwuchsmittel, zur Verwendung bei der Behandlung von Haarausfall, androgenetischer Alopezie, *Alopezia senilis, Alopezia areata,* bei der Vitalisierung von Haaren, bei der Anregung des Energiestoffwechsel in Haarfollikeln, bei der Aktivierung von Haarfollikeln, bei der Förderung oder Verstärkung des Haarwuchses, bei der Haarverdickung, bei der Beeinflussung der Keratinsynthese und bei der Haarkonditionierung.

Es ist daher vorgehen, das offenbarte Haarwuchsmittel zur Behandlung von Haarausfall jedweder Genese zu verwenden. Das Haarwuchsmittel regt somit die Neubildung von Haaren an, ohne sich negativ auf den vorhandenen Haarwuchs auszuwirken. Vielmehr nimmt der Erfinder an, dass sich das Haarwuchsmittel auch positiv auf das vorhandene Kopfhaar auswirkt.

Die Verwendung des Haarwuchsmittels erfolgt in einfacher Weise, so dass diese Anwendung im täglichen Lebensablauf keine negativen Einflüsse hervorruft. Die Anwendung ist einfach und sicher durchführbar.

Offenbart ist auch ein Haarwuchsmittel, zur Anwendung in einem Verfahren für die Behandlung von Haarausfall, androgenetischer Alopezie, *Alopezia senilis, Alopezia areata,* bei der Vitalisierung von Haaren, bei der Anregung des Energiestoffwechsels in Haarfollikeln, bei der Aktivierung von Haarfollikeln, bei der Förderung oder Verstärkung des Haarwuchses, bei der Haarverdickung, bei der Beeinflussung der Keratinsynthese und bei der Haarkonditionierung, dadurch gekennzeichnet, dass das Haarwuchsmittel auf die Haare und/oder die behaarte Haut und/oder die unbehaarte Haut aufgebracht wird.

Gegenstand ist somit auch ein Verfahren zur Behandlung von Haarausfall jedweder Genese durch Verwendung des Haarwuchsmittels, bei dem man das erfindungsgemäße Haarwuchsmittel auf der Kopfhaut aufträgt und einwirken lässt.

Besonders bevorzugt ist dabei die Anwendung, wobei nach dem Aufbringen des Haarwuchsmittels und einer Einwirkzeit von 5 bis 30 Minuten eine Pflegekomponente (zweite Komponente) aufgebracht wird, wobei die Pflegekomponente ein pflanzliches Öl ist, wobei das pflanzliche Öl ausgewählt ist aus Olivenöl, Sonnenblumenöl, Rapsöl, Distelöl, Leinöl, Sojaöl, Palmöl, Palmkernöl, Traubenkernöl, Arganöl, Kürbiskernöl, Mandelöl, Jojobaöl, Orangenöl, Weizenkeimöl, Pfirsichkernöl, den flüssigen Anteile des Kokosöls und deren Mischungen.

Insbesondere bevorzugt ist dabei ferner, dass die Pflegekomponente weiterhin elementaren Schwefel enthält.

Bevorzugt ist ferner eine Anwendung des Haarwuchsmittels, wobei das Aufbringen des Haarwuchsmittels derart erfolgt, dass man ein Kopfkissen, eine Kopfkissenauflage oder einen Kopfkissenbezug mit dem Haarwuchsmittel imprägniert und dadurch während der Nachtruhe die Einwirkzeit des Mittels auf der Kopfhaut verlängert und die Wirkung intensiviert.

Bevorzugt ist außerdem eine Anwendung des Haarwuchsmittels, wobei das Aufbringen des Haarwuchsmittels derart erfolgt, dass man eine enganliegende Mütze mit dem Haarwuchsmittel imprägniert und durch das Tragen dieser Mütze auch tagsüber die Einwirkzeit des Mittels verlängert und die Wirkung intensiviert.

Besonders vorteilhaft ist es, dass das Haarwuchsmittel auch mittels eines Kleidungsstücks wie einer Mütze oder über ein Kopfkissen auftragen lässt. Dadurch kann die Einwirkzeit verlängert und somit die Wirkung des Mittels verbessert werden.

Überraschenderweise wurde festgestellt, dass sich das Haarwachstum allein durch die Anwendung einer stark alkalischen wässrigen Lösung auf die kahlen Hauptstellen anregen lässt. Der pH-Wert sollte dabei in einem Bereich von 9 bis 12 liegen. Damit ist sichergestellt, dass keine Verätzungen an der Haut auftreten. Der pH-Wert des Haarwuchsmittels lässt sich durch die Verwendung einer leicht zu ermittelnden Menge eines Alkalisalzes in Form einer wässrigen Lösung herstellen.

Ferner ist die Herstellung des Haarwuchsmittels in einfacher und kostengünstiger Weise möglich.

Vorteilhafterweise werden im Rahmen der vorliegenden Offenbarung ausschließlich für den menschlichen Organismus bekanntermaßen unbedenkliche Wirkstoffe verwendet. Die Mittel enthalten keine weiteren chemischen Basis- sowie Zusatzstoffe oder Konservierungsstoffe. Lediglich eine zweite, pflegende Komponente könnte bei der Anwendung des Mittels vorteilhafterweise verwendet werden.

Die folgenden Beispiele erläutern die Erfindung, ohne sie jedoch darauf einzuschränken:

### Beispiel 1: Herstellung des Haarwuchsmittels

Zur Herstellung des Mittels werden Natriumcarbonat und aqua purificata verwendet. *Aqua purificata* und Natriumcarbonat werden in separate Behälter eingebracht. Das Aqua purificata wird stark erhitzt (Siedetemperatur mind. 98 Grad Celsius) und dann in das Gefäß mit Natriumcarbonat rasch eingebracht (Vorsicht: starke Wärme- und Dampfentwicklung). Anschließend wird die Mischung zum fertigen Produkt langsam abgekühlt.

Eine besonders reine Herstellung für das Haarwuchsmittel sieht vorzugsweise Natriumcarbonat (calciniert) und bidestilliertes Wasser vor. Bidestilliertes Wasser und Natriumcarbonat (calciniert) werden in separate Quarzglasbehälter eingebracht. Das bidestillierte Wasser wird stark erhitzt (Siedetemperatur mind. 98 Grad Celsius) und dann in das Quarzglasgefäß mit Natriumcarbonat (calciniert) rasch eingebracht (Vorsicht: starke Wärme- und Dampfentwicklung). Anschließend wird die Mischung zum fertigen Produkt langsam abgekühlt.

Eine besonders gut geeignete Herstellung für das Haarwuchsmittel sieht vorzugsweise Natriumcarbonat (calciniert) und VE-Wasser vor. VE-Wasser und Natriumcarbonat werden in separate Edelstahlgefäße eingebracht. Das VE-Wasser wird stark erhitzt (Siedetemperatur mind. 98 Grad Celsius) und dann in das Edelstahlgefäß mit Natriumcarbonat rasch eingebracht (Vorsicht: starke Wärme- und Dampfentwicklung). Anschließend wird die Mischung zum fertigen Produkt langsam abgekühlt.

Die hergestellte Lösung ist stark alkalisch (pH-Wert ca. 11,5) und nahezu geruchlos.

Die Flüssigkeit sollte in einem luftdichten Behältnis aufbewahrt werden, da sonst ein Zerfallsprozess einsetzt, der sich auf die Wirksamkeit des erfindungsgemäßen Haarwuchsmittels auswirken kann.

Das Mittel lässt sich in zwei Formen verwenden. Die erste ist flüssig, geeignet für die topische Anwendung und zur Imprägnierung, wie hierin beschrieben. Die zweite Form ist pulverförmig (weißes Pulver) und entsteht sowohl bei der Imprägnierung und anschließender Lufttrocknung und bleibt als kleine Teilchen wirksam im Gewebe der Kopfkissenbezüge, Frotteehandtücher, bzw. in den Mützen. Durch langsame Verdunstung der hergestellten Flüssigkeit kann das weiße Pulver ebenfalls erzeugt werden. Diese sehr konzentrierte Form kann ebenfalls zur äußeren Anwendung auf der Kopfhaut verwendet werden.

Es hat sich gezeigt, dass die direkte Verwendung der Pulverform des Mittels besonders gut auf haarfreien Bereichen aufzutragen ist. Das aber nur in sehr kleiner Menge und in einer sehr dünnen Schicht.

### Beispiel 2: Erfindungsgemässe Anwendung eines Haarwuchsmittels nach Beispiel 1 auf der Kopfhaut

Das Mittel wird topisch angewendet, indem es in einer zur Erzielung des gewünschten Effektes ausreichenden Menge auf die Kopfhaut aufgetragen und leicht einmassiert wird.

Für eine besonders geeignete Anwendung wird eine kleine Menge (2 ml bis 4 ml), bzw. mehrmals eine kleine Menge (insgesamt ca. 4 ml bis 10 ml) je nach Haarlänge und Haarausfallintensität von dem Mittel auf die Handinnenfläche aufgebracht und damit die betroffenen Stellen der Kopfhaut und danach die gesamte Kopfhaut massiert.

Für normal empfindliche Kopfhaut hat sich folgende Rezeptur als besonders geeignet erwiesen:
Natriumcarbonat (calc.) 6,0 mg
VE-Wasser 1000 mg

Für empfindliche Kopfhaut hat sich folgende erfindungsgemäße Rezeptur als besonders geeignet erwiesen:
Natriumcarbonat (calc.) 0,6 mg
VE-Wasser 1000 mg

Für sehr empfindliche Kopfhaut hat sich folgende Rezeptur als besonders geeignet erwiesen:
Natriumcarbonat (calc.) 0,006 mg
VE-Wasser 1000 mg

Eine optimale Wirksamkeit beruht auf einer 4-10x täglichen Anwendung. Bei der oben besonders gut geeigneten Rezeptur für normal-sensible Kopfhaut hat sich eine Dosierung von 6 x täglich als sehr vorteilhaft erwiesen.

### Beispiel 3: Weitere Zusammensetzung des Haarwuchsmittels

In der Tabelle 1 sind weitere Haarwuchsmittel angegeben. Die Herstellung der Mittel erfolgt in Analogie zum Beispiel 1.

**Tabelle 1**

| Alkalisalz | Menge [mg] | Wasserart | Menge [mg] |
|---|---|---|---|
| Na₂CO₃ (calc.) | 19,23 | VE-Wasser | 1000 |
| Na₂CO₃(calc.) | 15,23 | VE-Wasser | 1000 |
| Na₂CO₃ (calc.) | 10,00 | VE-Wasser | 1000 |

### Beispiel 4: Anwendung eines erfindungsgemäßen Haarwuchsmittels mit zusätzlicher zweiter Komponente

Eine erste Komponente (reaktive Komponente, hier: wässrige Lösung mit Na₂CO₃ wie im Beispiel 2 beschrieben) wird zunächst sanft auf die Kopfhaut aufgetragen und anschließend einmassiert - zuerst auf den kahlen Stellen, dann auch auf den Stellen mit noch vorhandenem Haar. Eine Reaktion mit der Kopfhaut setzt sofort ein und hält in Phase 1 - je nach Typ - gut 15 bis 20 Minuten an. Danach wird Komponente 2 (Pflegekomponente, hier: pflanzliches Öl und weiterer Wirkstoff) ebenfalls auf die Kopfhaut aufgetragen und sanft einmassiert. Komponente 2 bildet sofort einen leicht öligen und dadurch pflegenden Fettfilm, der die Kopfhaut sofort pflegt, während die erste Komponente weiterwirkt (Phase 2).

Bevorzugt sollte wie folgt vorgegangen werden: Nach dem morgendlichen Waschen der Haare und anschließendem gründlichen, aber sanftem Abtrocknen, kann die erste Komponente aufgetragen und einmassiert werden. Nach ca.15 - 20 Minuten kann die zweite Komponente aufgebracht und einmassiert werden. Beide Komponenten wirken - je nach Form der Bewegung (z.B. Schreibtisch oder Sport) - ca. ein bis vier Stunden. Anschließend sollte die Prozedur wiederholt werden (ohne Haare waschen). Bis zum Abend wird dies entsprechend wiederholt. Vor dem Schlafengehen sollte die letzte Prozedur durchgeführt werden. Am nächsten Morgen wird diese Vorgehensweise wiederholt, beginnend mit dem Waschen der Haare.

### Beispiel 5: Zusammensetzung und Herstellung der zweiten Komponente

Als zweite Komponente wird erfindungsgemäß ein fettes Öl oder Pflanzenöl verwendet. Im Sinne der Erfindung sind die Begriffe "fettes Öl", "Pflanzenöl" und "pflanzliches Öl" gleichwertig und werden hierin synonym verwendet. Geeignet ist jede Art von Pflanzenöl, das hautverträglich ist. Besonders bevorzugt sind Olivenöl, Sonnenblumenöl, Rapsöl, Distelöl, Leinöl, Sojaöl, Palmöl, Palmkernöl, Traubenkernöl, Arganöl, Kürbiskernöl, Mandelöl, Jojobaöl, Orangenöl, Weizenkeimöl, Pfirsichkernöl, den flüssigen Anteile des Kokosöls und deren Mischungen. Die Verwendung als zweite Komponente kann ohne weitere Zusätze direkt auf der Haut oder auf den Haaren aufgetragen werden.

Besonders bevorzugt ist es, wenn dem fetten Öl ein weiterer Bestandteil zugesetzt ist. Dieser weitere Bestandteil ist bevorzugt elementarer Schwefel.

In einer Ausführungsform umfasst die Konzentration 0,005 bis 10 Gew%, bevorzugt 0,05 bis 5 Gew%, insbesondere bevorzugt 0,1 bis 1,0 Gew% Schwefel (S), bei 100 Gew% Öl ausgewählt aus einer Gruppe der fetten Öle.

Eine bevorzugte Rezeptur für die zweite Komponente ist wie folgt zusammengesetzt:
Sonnenblumenöl 100,0 g
Schwefel 000,10 g

Eine weitere bevorzugte Rezeptur für die zweite Komponente ist wie folgt zusammengesetzt:
Olivenöl 100,0 g
Schwefel 000,20 g

Die Herstellung der zweiten Komponente wird wie folgt durchgeführt: Schwefel und mindestens ein Öl ausgewählt aus einer Gruppe der fetten Öle werden in separate Behälter eingebracht. Das ausgewählte Öl wird auf ca. 40 Grad Celsius leicht erhitzt. Anschließend wird das erwärmte Öl in das Gefäß mit dem Schwefel eingebracht und ca. 10 Minuten lang langsam verrührt. Anschließend wird die Mischung abgekühlt und vorhandener Bodensatz, sofern vorhanden (Schwefel), herausgefiltert.

Die zweite Komponente besitzt eine relativ hohe Viskosität, ist stark fettend (feuchtigkeitsspendend) sowie haut- und haarpflegend. Sie zeigt einen leichten Geruch des jeweils verwendeten Öls. Je nach Mischung kann auch ein sehr leichter Schwefelgeruch wahrnehmbar sein.

### Beispiel 6: Anwendung der zweiten Komponente

Die zweite Komponente wird topisch angewendet, indem diese in einer zur Erzielung des gewünschten Effektes ausreichenden Menge auf die Kopfhaut aufgetragen und leicht einmassiert wird. Für eine besonders geeignete Anwendung wird eine kleine Menge (0,5 ml bis 1 ml), bzw. mehrmals eine kleine Menge (insgesamt ca. 1 ml bis 2 ml) der zweiten Komponente, je nach Haarlänge und Haarausfallintensität, auf die Handinnenfläche aufgebracht und damit auf die betroffenen Stellen der Kopfhaut und danach die gesamte Kopfhaut damit massiert und so "befeuchtet".

### Beispiel 7: Imprägnierung von Frottee-Stoffen und Mützen mit dem Mittel

Für eine besonders geeignete Imprägnierung von Frottee-Stoffen wird einer normalen Waschmaschinen-Wäsche (z.B. 10 Frottee-Handtücher/ -Kopfkissenbezüge, normales Waschmittel, normale Waschmittelmenge, 60 °C) das Mittel, welches mindestens 1,5 Gew.-% Alkalisalz enthält, in das Weichspülerfach (100 bis 150 ml) gegeben. Dabei hat sich bewährt, die Schleuderdrehzahl auf max. 400 Umdrehungen/min zu begrenzen. Anschließend werden die Frottee-Handtücher bzw. die 10 Frottee-Kopfkissenbezüge normal an der Luft getrocknet. Nicht bewährt haben sich Wäschetrockner. Sowohl Wäschetrockner, als auch höhere Schleuderdrehzahlen können den Imprägnierungsgrad deutlich verschlechtern und so die gewünschte topische Einwirkung über die Kopfhaut stark reduzieren.

Für eine besonders geeignete Imprägnierung von Mützen wird einer normalen Waschmaschinen-Wäsche (10 bis 15 Mützen, normales Fein- oder Wollwaschmittel, normale Waschmittelmenge, 30 bis 60 °C (je nach Mützenstoff) anstatt des Weichspülers (100 bis 150 ml), das Mittel, welches mindestens 1,5 Gew.-% Natriumcarbonat enthält, eingefüllt. Dabei hat sich bewährt, die Schleuderdrehzahl auf max. 400 Umdrehungen/min zu begrenzen. Anschließend werden die Mützen normal an der Luft getrocknet. Nicht bewährt haben sich Wäschetrockner. Sowohl Wäschetrockner, als auch höhere Schleuderdrehzahlen können den Imprägnierungsgrad deutlich verschlechtern und so die gewünschte topische Einwirkung über die Kopfhaut stark reduzieren.

### Beispiel 8: Ergebnisse der regelmäßigen Anwendung der Mittel

Es wurde ein Mittel gemäß Beispiel 2 verwendet, das 6,0 mg Natriumcarbonat (calc.) in 1000 mg VE-Wasser enthielt. Der pH-Wert liegt bei ca. 11,5. Die Anwendung erfolgt wie im Beispiel 4 beschrieben.

Nach einiger Zeit ist folgendes zu beobachten:
- Steigerung und Aktivierung der Haarfollikel-Synthese
- Verlängerung der Wachstumsphase
- Steigerung der Haardicke
- Steigerung der Haardichte
- Vitalisierung des Haares

### Allgemein:

Je nach individueller Ursache und regelmäßiger Anwendung ist nach ca. 12 bis 15 Monaten eine bereits leicht sichtbare, nach 24 Monaten eine deutlich sichtbare Verbesserung des Gesamtbildes erkennbar.

### Im Speziellen:

Nach ca. 6 Monaten stellte sich eine sichtbare Stärkung der Flaumhaare ein, wobei diese unterschiedlich schnell weiterwuchsen. An kahlen Stellen war besonders gut zu beobachten, dass 60 bis 80 % dieser wachsenden Flaumhaare allerdings immer wieder ausfielen, bzw. nicht weiter wuchsen, so dass erst nach mehreren Generationen/Zyklen, zeitlich nach ca. 18 Monaten, insgesamt deutlich mehr längere und dickere Haare zu erkennen waren, die zum Teil nicht mehr von den vorher vorhandenen Haaren zu unterscheiden waren. Haarwuchs auf nicht kahlen Flächen wurde insgesamt dichter. Nach ca. 24 bis 36 Monaten waren die vormals haarlosen Stellen wieder (nahezu) mit lockeren Haaren bedeckt. Die Revitalisierung/Reaktivierung des Haarwuchses stellte sich vom Haarkranz zur Mitte des Kopfes nach oben und in Richtung Schläfe bzw. Hinterkopf, quasi in Umkehrung der Glatzenbildung ein. Am längsten wenig behaart blieb der Wirbel am Hinterkopf. Bis die Haare insgesamt wieder so dicht sind, dass man die Kopfhaut fast nicht mehr sieht, ist, nach bisheriger Beobachtung, mit ca. 60 bis 80 Monate zu rechnen. In einem Beobachtungsbeispiel wurde das Hamilton-Norwood-Schema herangezogen. Vor dem Beginn der Behandlung lag ein Typ VI vor, nach 28 Monaten lag ein Typ V vor. Es wird erwartet, dass nach ca. 60 Monaten Typ III bis Typ II vorliegen könnte.

Überraschenderweise wurde festgestellt, dass das stark alkalische Haarwuchsmittel die Kopfhaut nicht sonderlich irritierte, weder an den kahlen Stellen, noch am und im Haarkranz. Ab und zu stellte sich ein leichtes Kribbeln auf der Kopfhaut ein, kurz nachdem das erfindungsgemäße Haarwuchsmittel aufgetragen wurde. Vermutlich ist das auf die stark feuchtigkeitsentziehende Wirkung des erfindungsgemäßen Haarwuchsmittels zurückzuführen. Bei normal sensitiver Kopfhaut verschwand das Kribbeln oftmals wieder durch leichtes "durch die Haare fahren" mit einer oder beiden Händen.

Als besonders geeignet stellte sich heraus, insbesondere bei empfindlicher und sehr empfindlicher Kopfhaut, dass leichtes Einmassieren einer handelsüblichen Feuchtigkeitscreme bei Auftreten dieser Hautirritation diese häufig sofort wiedervollständig beseitigen konnte. Es hat sich gezeigt, dass es empfehlenswert sein kann, vor der ersten Anwendung des Mittels die Empfindlichkeit der Kopfhaut gegenüber dem erfindungsgemäßen Mittel zu testen. Bewährt hat sich hierbei, nicht direkt mit der topischen Anwendung auf der Kopfhaut zu beginnen, sondern über einen Zeitraum von ca. 10-14 Tagen die indirekte Einwirkung durch Verwendung imprägnierter Kopfkissenbezüge zu testen.

Folgende Imprägnierungslösung hat sich hierbei besonders bewährt:
Natriumcarbonat (calc.) 15,23 mg
VE-Wasser 1000 mg

Es hat sich gezeigt, dass der Einsatz eines Mittels als topische Anwendung dann sinnvoll ist, wenn während des Versuchszeitraumes weder eine Rötung der Kopfhaut, noch ein Kribbeln auf dem Kopf aufgetreten ist.

## Patentansprüche

1. Haarwuchsmittel, bestehend aus einer wässrigen Natriumcarbonatlösung, mit einem pH-Wert der Lösung im Bereich von 9,5 bis 12, zur Verwendung bei der Förderung oder Verstärkung des Haarwuchses und bei der Haarverdickung, wobei man die wässrige Lösung auf der Kopfhaut aufbringt und einwirken lässt.

2. Haarwuchsmittel zur Verwendung, gemäß Anspruch 1, **dadurch gekennzeichnet, dass** der pH-Wert zwischen 9,5 und 11,9, bevorzugt zwischen 10 und 11,7 und besonders bevorzugt zwischen 10,0 und 11,5 beträgt.

3. Haarwuchsmittel zur Verwendung, gemäß Anspruch 1 oder 2, **dadurch gekennzeichnet, dass** der Gehalt an Natriumcarbonat 0,00005 bis 5 Gew.-%, bevorzugt 0,0005 bis 2 Gew.-% und besonders bevorzugt 0,0005 bis 1 Gew.-% beträgt.

4. Haarwuchsmittel zur Verwendung, gemäß mindestens einem der voranstehenden Ansprüche **dadurch gekennzeichnet, dass** das Wasser ausgewählt ist aus der Gruppe enthaltend *aqua purificata,* destillatgleiches Wasser, bidestilliertes Wasser, VE-Wasser oder demineralisiertes Wasser.

5. Haarwuchsmittel zur Verwendung, gemäß mindestens einem der voranstehenden Ansprüche **dadurch gekennzeichnet, dass** nach dem Aufbringen der wässrigen Lösung und einer Einwirkzeit von 5 bis 30 Minuten eine Pflegekomponente aufgebracht wird, wobei die Pflegekomponente ein pflanzliches Öl ist, wobei das pflanzliche Öl ausgewählt ist aus Olivenöl, Sonnenblumenöl, Rapsöl, Distelöl, Leinöl, Sojaöl, Palmöl, Palmkernöl, Traubenkernöl, Arganöl, Kürbiskernöl, Mandelöl, Jojobaöl, Orangenöl, Weizenkeimöl, Pfirsichkernöl, den flüssigen Anteile des Kokosöls und deren Mischungen.

6. Haarwuchsmittel zur Verwendung, gemäß Anspruch 5, **dadurch gekennzeichnet, dass** die Pflegekomponente weiterhin elementaren Schwefel enthält.

7. Haarwuchsmittel zur Verwendung, gemäß mindestens einem der Ansprüche 1-4, **dadurch gekennzeichnet, dass** das Aufbringen der wässrigen Lösung derart erfolgt, dass man ein Kopfkissen, eine Kopfkissenauflage oder einen Kopfkissenbezug mit der Lösung imprägniert und dadurch während der Nachtruhe die Einwirkzeit des Mittels auf der Kopfhaut verlängert und die Wirkung intensiviert.

8. Haarwuchsmittel zur Verwendung, gemäß Anspruch 7, **dadurch gekennzeichnet, dass** das Aufbringen der wässrigen Lösung derart erfolgt, dass man eine enganliegende Mütze mit der wässrigen Lösung imprägniert und durch das Tragen dieser Mütze auch tagsüber die Einwirkzeit des Mittels verlängert und die Wirkung intensiviert.

## Claims

1. Hair restorer, consisting of an aqueous sodium carbonate solution with the pH value of the solution in the range of 9.5 to 12, for use in the promotion or strengthening of hair growth and in hair thickening, wherein the aqueous solution is applied on the scalp and is allowed to take action.

2. Hair restorer for use according to claim 1, **characterized in that** the pH value is between 9.5 and 11.9, preferred between 10 and 11.7, and especially preferred between 10.0 and 11.5.

3. Hair restorer for use according to claim 1 or 2, **characterized in that** the content of sodium carbonate is 0.00005 to 5 weight-%, preferred 0.0005 to 2 weight-%, and especially preferred 0.0005 to 1 weight-%.

4. Hair restorer for use according to at least one of the preceding claims, **characterized in that** the water is selected from the group containing *aqua purificata,* distillate-like water, double-distilled water, deionized water, or demineralized water.

5. Hair restorer for use according to at least one of the preceding claims, **characterized in that** after the aqueous solution has been applied and an action time of 5 to 30 minutes, a care compound is applied, the care compound being a vegetable oil, the vegetable oil being selected from olive oil, sunflower oil, rapeseed oil, safflower oil, linseed oil, soybean oil, palm oil, palm kernel oil, grape seed oil, argan oil, pumpkin seed oil, almond oil, jojoba oil, orange oil, wheat germ oil, peach kernel oil, the liquid components of coconut oil and their mixtures.

6. Hair restorer for use according to claim 5, **characterized in that** the care component also contains elemental sulfur.

7. Hair restorer for use according to at least one of the claims 1-4, **characterized in that** the application of the aqueous solution is performed in such a way that a pillow, pillow pad or pillowcase is impregnated with the solution, and thereby during sleep lengthening the action time of the agent on the scalp and intensifying the effect.

8. Hair restorer for use according to claim 7, **characterized in that** the application of the aqueous solution is performed in such a way that a tight-fitting cap is impregnated with the aqueous solution and by wearing this cap during the day the exposure time of the agent is extended and the effect is intensified.

## Revendications

1. Produit pour la croissance des cheveux consistant en une solution aqueuse de carbonate de sodium, avec un pH de la solution allant de 9,5 à 12, pour une utilisation dans le renforcement ou l'amélioration de la croissance des cheveux et dans l'épaississement des cheveux, la solution aqueuse étant appliquée sur le cuir chevelu et étant laissée afin d'agir.

2. Produit pour la croissance des cheveux pour une utilisation selon la revendication 1, **caractérisé en ce que** le pH est compris entre 9,5 et 11,9, de préférence entre 10 et 11,7 et, de façon particulièrement préférée, entre 10,0 et 11,5.

3. Produit de croissance des cheveux pour une utilisation selon la revendication 1 ou la revendication 2, **caractérisé en ce que** la teneur en carbonate de sodium est de 0,00005 à 5 % en poids, de préférence de 0,0005 à 2 % en poids et, de façon particulièrement préférée, de 0,0005 à 1 % en poids.

4. Produit de croissance des cheveux pour une utilisation selon au moins une des revendications précédentes, **caractérisé en ce que** l'eau est choisie dans le groupe comprenant l*'aqua purificata,* l'eau distillée, l'eau doublement distillée, l'eau dé-ionisée ou l'eau déminéralisée.

5. Produit de croissance des cheveux pour une utilisation selon au moins une des revendications précédentes, **caractérisé par le fait qu'**après l'application de la solution aqueuse et un temps de contact de 5 à 30 minutes, un composant de soin est appliqué, le composant de soin étant une huile végétale, l'huile végétale étant sélectionnée parmi l'huile d'olive, huile de tournesol, huile de colza, huile de carthame, huile de lin, huile de soja, huile de palme, huile de palmiste, huile de pépins de raisin, huile d'argan, huile de pépins de courge, huile d'amande, huile de jojoba, huile d'orange, huile de germe de blé, huile de noyau de pêche, les parties liquides de l'huile de noix de coco et les mélanges de celles-ci.

6. Produit de croissance des cheveux pour une utilisation selon la revendication 5, **caractérisé en ce que** le composant de soin comprend en outre du soufre élémentaire.

7. Produit de croissance des cheveux pour une utilisation selon au moins l'une des revendications 1 à 4, **caractérisé en ce que** l'application de la solution aqueuse est effectuée de telle sorte qu'un oreiller, un coussin ou une taie d'oreiller soit imprégné de la solution, ce qui prolonge le temps d'action du produit sur le cuir chevelu pendant le repos nocturne et intensifie l'effet.

8. Produit de croissance des cheveux pour une utilisation selon la revendication 7, **caractérisé en ce que** l'application de la solution aqueuse est effectuée de telle manière qu'un bonnet soit imprégné de la solution aqueuse, le port de ce bonnet pendant la journée prolongeant la durée d'exposition au produit est prolongée et intensifiant l'effet.
